# FASCICULE DE BREVET EUROPEEN

(11) **EP 2 512 415 B1**
(45) Date de publication et mention de la délivrance du brevet: **02.08.2017**
(21) Numéro de dépôt: 10805802.5
(22) Date de dépôt: 14.12.2010
(51) Int. Cl.: A61K 8/34, A61K 8/46, A61K 8/73, A61K 8/81, A61K 8/87, A61K 8/36, A61Q 5/02, A61Q 5/12

(54) **COMPOSITION COSMETIQUE COMPRENANT UN TENSIOACTIF, UN ALCOOL GRAS LIQUIDE ET UN POLYMERE ASSOCIATIF NON IONIQUE ET PROCÉDÉ DE TRAITEMENT COSMÉTIQUE**
KOSMETISCHE ZUSAMMENSETZUNG MIT EINEM TENSID, EINEM FLÜSSIGEN FETTALKOHOL UND EINEM NICHTIONISCHEN ASSOZIATIVEN POLYMER SOWIE KOSMETISCHES BEHANDLUNGSVERFAHREN DAMIT
COSMETIC COMPOSITION COMPRISING A SURFACTANT, A LIQUID FATTY ALCOHOL AND A NON-IONIC ASSOCIATIVE POLYMER AND COSMETIC TREATMENT METHOD

(30) Priorité: 17.12.2009 FR 0959124; 23.12.2009 US 289632 P
(43) Date de publication de la demande: 24.10.2012
(73) Titulaire: L'Oréal, 75008 Paris (FR)
(72) Inventeur: MATHONNEAU, Estelle, F-75010 Paris (FR)
(74) Mandataire: Dodin, Catherine
(86) Numéro de dépôt international: PCT/FR2010/052705
(87) Numéro de publication internationale: WO 2011/073564

(56) Documents cités:
- FR-A1- 2 848 105
- US-B1- 6 218 345

## Description

La présente invention concerne une composition de lavage et de conditionnement des matières kératiniques, en particulier des fibres kératiniques, comprenant un tensioactif anionique particulier, un alcool gras liquide et un polymère épaississant non ionique particulier, l'utilisation de ladite composition pour le lavage et le conditionnement des matières kératiniques, en particulier des fibres kératiniques, ainsi qu'un procédé de mise en oeuvre de ladite composition.

Dans le domaine des shampoings conditionneurs, on associe généralement une base lavante et un agent de conditionnement qui peut être un polymère cationique, un polymère amphotère, une silicone, une huile synthétique ou naturelle, un corps gras ou un de leurs mélanges. Ces agents de conditionnement sont utilisés pour améliorer le démêlage et la douceur des cheveux mouillés et séchés mais peuvent avoir tendance à alourdir la chevelure et à la ternir.

L'utilisation d'agents conditionneurs insolubles est fortement limitée :
- d'une part, du fait des difficultés de stabilisation dans des compositions détergentes, si on veut maintenir le niveau des qualités d'usage (stabilité, pouvoir moussant, démarrage de mousse),
- d'autre part, du fait des défauts cosmétiques en termes d'alourdissement, de charge et de regraissage liés à des dispersions grossières ou hétérogènes dans ces mêmes compositions détergentes.

Les agents de conditionnement insolubles, en particulier les alcools gras liquides, sont connus et utilisés dans les compositions de shampooings comme notamment dans les documents JP2002-20791, JP9-30938, US2009/005449 et US2009/005460.

Cependant, ces compositions ne sont pas épaissies et/ou stables et/ou ne présentent pas des performances cosmétiques de haut niveau en termes de démêlage et de lissage, sans charge ni alourdissement de la chevelure et en maintenant le niveau des qualités d'usage.

En effet, on recherche des produits épaissis que l'on peut doser et prendre aisément dans la main. Pour ce faire, ces produits doivent présenter une certaine consistance ou viscosité. En effet, un produit liquide est beaucoup plus difficile à doser et s'écoule facilement entre les doigts.

Pour épaissir les compositions lavantes, on a déjà utilisé des polymères naturels tels que les celluloses. Malheureusement, ces épaississants donnent des compositions instables et/ou qui ne sont pas lisses et homogènes. De plus, ces épaississants présentent l'inconvénient de diminuer la qualité de la mousse et les performances cosmétiques des shampooings, notamment en rendant les cheveux plus chargés et plus rêches. La mousse des compositions épaissies n'est généralement pas suffisamment douce, elle ne se développe pas facilement que ce soit en vitesse ou en abondance.

Ainsi, il subsiste le besoin d'un système épaississant permettant d'épaissir, de manière convenable une composition lavante comprenant un alcool gras liquide sans influer sur les propriétés cosmétiques et moussantes desdites compositions ou en les améliorant.

La demanderesse a maintenant découvert que l'utilisation de polymère associatif non ionique particulier dans des compositions comprenant un tensioactif anionique particulier et un alcool gras liquide permettrait de surmonter les inconvénients indiqués ci-dessus, et d'obtenir ainsi une composition stable d'aspect homogène et esthétique. De plus, les compositions présentent une bonne abondance de mousse. Par ailleurs, les propriétés cosmétiques et en particulier le lissage, la douceur, la souplesse, la brillance sont améliorées. Dans le cas des cheveux frisés et/ou crépus, on observe une diminution du volume permettant un meilleur contrôle de la chevelure.

Les compositions selon l'invention apportent également une protection de la couleur aux lavages des cheveux artificiellement colorés.

L'invention a donc pour objet une composition notamment de lavage et de conditionnement des matières kératiniques, en particulier des fibres kératiniques, comprenant dans un milieu cosmétiquement acceptable,
- de 3 à 50% en poids par rapport au poids total de la composition d'un ou plusieurs tensioactifs anioniques,
- un ou plusieurs alcools liquides en C₁₀-C₃₀ choisis parmi les alcools en C₁₀-C₃₀ ramifiés et /ou insaturés, et
- un ou plusieurs polymères associatifs non ioniques,
la composition comprenant un ou plusieurs tensioactifs an ioniques choisis parmi les alkylsulfates, et les alkyléthersulfates, en particulier sous forme de sels de métaux alcalins ou alcalino-terreux, d'ammonium, d'amine ou d'aminoalcool ; et les acides (alkyl en C₆₋₂₄)éther-carboxyliques polyoxyalkylénés et leurs sels, en particulier ceux comportant de 2 à 50 motifs oxyde d'éthylène; et ladite composition comprenant un ou plusieurs polymères associatifs non ioniques choisis parmi, seuls ou en mélanges :
- (1) les celluloses modifiées par des groupements comportant au moins une chaîne grasse comportant au moins 8 atomes de carbone; et
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

Un autre objet de l'invention est l'utilisation de ladite composition pour le lavage et le conditionnement des matières kératiniques, en particulier des fibres kératiniques, et plus particulièrement des cheveux.

L'invention a encore pour objet un procédé de lavage et de conditionnement des fibres kératiniques mettant en oeuvre la composition selon l'invention.

D'autres objets, caractéristiques, aspects et avantages de l'invention apparaîtront encore plus clairement à la lecture de la description et des divers exemples qui suivent.

Dans la présente demande, on qualifie une entité comme étant "anionique" lorsqu'elle possède au moins une charge négative permanente ou lorsqu'elle peut être ionisée en une entité chargée négativement, dans les conditions d'utilisation des compositions de l'invention (milieu, pH par exemple) et ne comprenant pas de charge cationique.

On qualifie une entité comme étant "non ionique" lorsqu'elle n'est ni cationique ni anionique au sens de la présente demande, en particulier elle ne comporte aucun groupe cationique ou anionique au sens de la présente demande.

Par "cosmétiquement acceptable" ou "physiologiquement acceptable", on entend qui est compatible avec l'application sur le corps d'un être vivant, en particulier le corps humain, et notamment son cuir chevelu et ses cheveux.

Les tensioactifs anioniques utilisés dans la composition sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les aikyléthersulfates; les groupes alkyle de tous ces composés comportant de 6 à 24 atomes de carbone.

En outre, on peut encore citer les acides (alkyl en C₆₋₂₄)éther-carboxyliques polyoxyalkylénés, et leurs sels, en particulier ceux comportant de 2 à 50 motifs oxyde d'éthylène.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

Le ou les tensioactifs anioniques sont présents en une quantité totale allant de 3 à 50 % en poids, mieux encore de 4 à 20% en poids par rapport au poids total de la composition.

Les« alcools gras liquides » sont liquides à température ambiante (25°C) et à pression atmosphérique (1 atm), et insolubles dans l'eau (c'est-à-dire, présente une solubilité dans l'eau inférieure à 1 % en poids et de préférence inférieure à 0,5 % en poids), et sont solubles, dans les mêmes conditions de température et de pression, dans au moins un solvant organique (par exemple l'éthanol, le chloroforme, le benzène ou l'huile de vaseline) à au moins 1 % en poids.

Les alcools gras liquides, en particulier ceux en C₁₀-C₃₀ présentent des chaînes carbonées ramifiées ou possèdent une ou plusieurs (de préférence 1 à 3) insaturations.

Les alcools gras selon l'invention sont de préférence ramifiés et/ou insaturées, et comportent de 12 à 40 atomes de carbone. Les alcools gras de l'invention sont non oxyalkylénés et non glycérolés.

Les alcools gras liquides présentent de préférence la structure R-OH, dans laquelle R désigne de préférence un groupement alkyle ramifié en C₁₂-C₂₄ ou alkényle en C₁₂-C₂₄. R peut être substitué par un ou plusieurs groupements hydroxy. De préférence, R ne contient pas de groupement hydroxy.

A titre d'exemple on peut citer l'alcool oléique, l'alcool linoléique l'alcool linoléique, l'alcool isocétylique, l'alcool isostéarylique, le 2-octyl-1-dodécanol, le 2-butyl octanol, le 2-hexyl-1-décanol, le 2-decyl-1-tétradécanol, le 2-tétradécyl-1-cétanol et leur(s) mélange(s).

De préférence l'alcool gras liquide de l'invention est un alcool saturé ramifié. Encore plus préférentiellement l'alcool gras liquide de l'invention est le 2-octyl 1-dodécanol.

Les alcools gras peuvent être des mélanges, ce qui signifie que dans un produit commercial peuvent coexister plusieurs espèces notamment de longueurs de chaînes différentes, sous forme d'un mélange.

Le ou les alcools gras liquides de l'invention sont généralement présents en une quantité allant de 0,01 à 10 % en poids, préférentiellement de 0,1 à 5% en poids et, mieux encore, de 0,5 à 3% en poids par rapport au poids total de la composition.

Par composition épaissie on entend, au sens de la présente invention une composition présentant une viscosité d'au moins 25 cps et de préférence d'au moins 50 cps à la température de 25°C et avec un taux de cisaillement de 1s-1. Ces viscosités sont mesurables avec un rhéomètre à géométrie cone-plan.

La composition comprend par ailleurs un ou plusieurs polymères associatifs non ioniques particuliers.

La notion de polymère au sens de la présente invention n'inclut pas les esters d'acides gras et de polyalkylèneglycols, les alcools gras polyoxyalkylénés.

Par « polymères associatifs » on entend des polymères dont les molécules sont capables dans un milieu donné de préférence aqueux, de s'associer réversiblement entre elles ou avec d'autres molécules. Leur structure chimique comprend au moins une zone hydrophile et au moins une zone hydrophobe, cette dernière étant caractérisée par la présence d'au moins une chaîne grasse comportant au moins 8 atomes de carbone, de préférence de 8 à 30 atomes de carbone.

Les polymères conformes à l'invention ont en général une masse molaire en poids allant de 5 000 à 10 000 000, plus préférentiellement de 50 000 à 8 000 000 et encore plus préférentiellement de 100 000 à 3 000 000.

Les polymères associatifs non ioniques sont choisis parmi :
- (1) les celluloses modifiées par des groupements comportant au moins une chaîne grasse ;
pour lesquelles peut citer à titre d'exemples :
- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C8-C22, comme le produit NATROSOL PLUS GRADE 330 CS® (alkyles en C16) vendu par la société AQUALON, ou le produit BERMOCOLL EHM 100® vendu par la société BEROL NOBEL,
- (6) les polyuréthanes polyéthers comportant dans leur chaîne, à la fois des séquences hydrophiles, de nature le plus souvent polyoxyéthylénée, et des séquences hydrophobes qui peuvent être par exemple des enchaînements aliphatiques seuls et/ou des enchaînements cycloaliphatiques et/ou aromatiques.

De préférence, les polyéthers polyuréthanes comportent au moins deux chaînes lipophiles hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

En particulier, il est possible qu'une ou plusieurs chaînes pendantes soient prévues.

En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non ioniques comportent une liaison uréthanne entre les séquences hydrophiles, d'où l'origine du nom.

Par extension figurent aussi parmi les polyéthers polyuréthanes non ioniques à chaîne grasse, ceux dont les séquences hydrophiles sont liées aux séquences lipophiles par d'autres liaisons chimiques.

A titre d'exemples de polyéthers polyuréthanes non ioniques à chaîne grasse utilisables dans l'invention, on peut aussi utiliser aussi le Rhéolate 205® à fonction urée vendu par la société RHEOX ou encore les Rhéofates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX.

Plus particulièrement encore, selon l'invention, on peut aussi utiliser un polyéther polyuréthane susceptible d'être obtenu par polycondensation d'au moins trois composés comprenant (i) au moins un polyéthylèneglycol comprenant de 120 à 180 moles d'oxyde d'éthylène, (ii) de l'alcool gras en C10-C20, en particulier de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Selon une forme particulière de l'invention, on utilisera un polyéther polyuréthane susceptible d'être obtenu par polycondensation d'au moins trois composés comprenant (i) au moins un polyéthylèneglycol comprenant de 100 à 180 moles d'oxyde d'éthylène, en particulier de 120 à 160 moles d'oxyde d'éthylène, (ii) un alcool gras en C16- 20 polyoxyéthyléné de 80 à 120 moles d'oxyde d'éthylène, en particulier 90 à 110 moles et notamment un alcool stéarylique polyoxyéthyléné comprenant de 80 à 120 moles d'oxyde d'éthylène, en particulier 90 à 110 moles et (iii) un diisocyanate aliphatique en particulier hexaméthylène diisocyanate.

De tels polyéther polyuréthanes sont vendus notamment par la société ELEMENTIS sous l'appellation RHEOLATE FX 1100 ^{®} qui est un polycondensat de polyéthylèneglycol à environ 136 moles d'oxyde d'éthylène, d'alcool stéarylique polyoxyéthyléné à environ 100 moles d'oxyde d'éthylène et de hexaméthylène diisocyanate (HDI) ayant un poids moléculaire moyen en poids de 25000 à 35000 (nom INCI : PEG-136/Steareth-100/HDI Copolymer).

Avantageusement, le ou les polymères associatifs non ioniques sont généralement présents en une quantité allant de 0,1 à 5 % en poids, de préférence de 0,5 à 4 % en poids,préférentiellement de 1,5 à 4% en poids, mieux de 1,5 à 3% en poids par rapport au poids total de la composition.

Dans une variante de l'invention le rapport pondéral polymère(s) associatif(s) non ioniques(s) / alcool(s) gras liquide(s) va de 0,005 à 50 de préférence de 0,1 à 30, mieux de 0,2 à 10 et encore plus particulièrement de 0,5 à 5.

La composition peut également comprendre en outre au moins un tensioactif non ionique et/ou au moins un tensioactif amphotère.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Parmi les tensioactifs non-ioniques cités ci-dessus, on utilise de préférence les (alkyl en C₆₋₂₄)polyglycosides, et plus particulièrement les (alkyl en C₈₋₁₈)polyglycosides.

Les agents tensioactifs amphotères, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₂₋₈)bétaïnes ou les (alkyl en C₈₋₂₀)amido(alkyl en C₂₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle saturé ou insaturé en C7-C23, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères cités ci-dessus, on utilise de préférence les (alkyl en C₈₋₂₀)-bétaïnes, les (alkyl en C₈₋₂₀)-amido(alkyl en C₂₋₈)bétaïnes, les alkylamphodiacétates et leurs mélanges.

Les tensioactifs non ioniques et/ou amphotères sont de préférence présents dans la composition selon l'invention en une quantité allant de 0 à 20 % en poids, mieux encore de 0,5 à 10% en poids par rapport au poids total de la composition.

La quantité totale de tensioactifs, est de préférence comprise entre 4 et 50 % en poids, mieux encore entre 5 et 30 % en poids par rapport au poids total de la composition.

La composition selon l'invention peut notamment comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 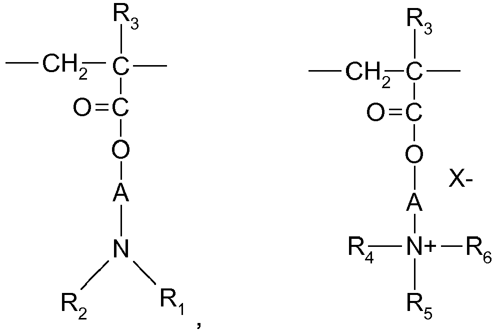 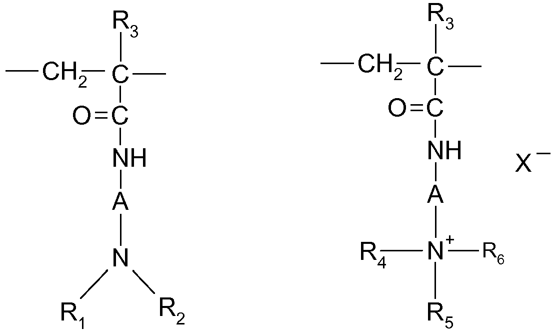 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES, - les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyl-triméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - les copolymères vinylpyrrolidone / méthacrylamide de diméthylamino-propyle quaternisé tel que le produit vendu sous la dénomination "GAFQUAT HS 100" par la société ISP,- les polymères de préférence réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "UCARE POLYMER JR" (JR 400 LT, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (II) : 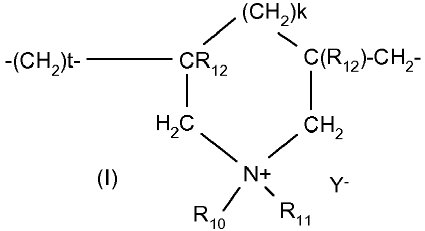 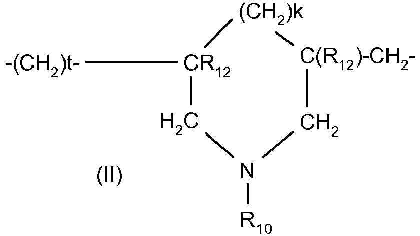 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de sels (par exemple chlorure) de diméthyldiallylammonium par exemple vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide commercialisés notamment sous la dénomination "MERQUAT 550" ou »MERQUAT 7SPR ».
(8) les polymères de diammonium quaternaire comprenant des motifs récurrents répondant à la formule : 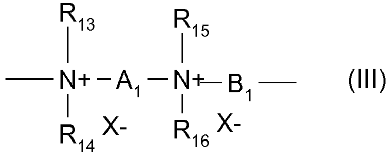 formule (III) dans laquelle :
   R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

   De préférence, X- est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 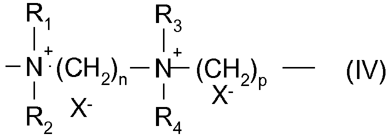 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
   Un composé de formule (IV) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires comprenant des motifs de formule (V): 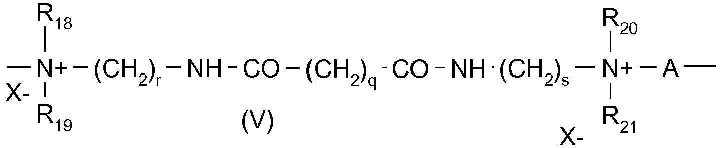 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)pOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogènure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination « JR 400 » par la Société AMERCHOL, les cyclopolymères cationiques, en particulier les homopolymères ou copolymères de sels (par exemple chlorure) de diméthyldiallylammonium, vendus sous les dénominations « MERQUAT 100 », « MERQUAT 550 » et « MERQUAT S » par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20%, de préférence de 0,05 à 10% et plus particulièrement de 0,1 à 5 % en poids par rapport au poids total de la composition.

La composition selon l'invention peut notamment comprendre en outre au moins une silicone. Cette silicone peut être linéaire, ramifiée ou cyclique, volatile ou non, organomodifiée ou non,

Par milieu cosmétiquement acceptable, on entend un milieu compatible avec les matières kératiniques, notamment les fibres kératiniques telles que les cheveux, mais aussi d'odeur, d'aspect et de toucher agréables. Le milieu est de préférence aqueux.

Le milieu cosmétiquement acceptable est constitué d'eau ou d'un mélange d'eau et d'au moins un solvant organique choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols.

De préférence, la composition comprend de 70 à 95 % en poids d'eau par rapport au poids total de la composition.

Le pH des compositions selon l'invention est généralement compris entre 2 et 11, de préférence entre 3 et 10 et, mieux encore entre 4 et 8.

La composition selon l'invention peut comprendre en outre des additifs choisis parmi les polymères anioniques, les polymères non ioniques, les polymères amphotères, les épaississants polymères non associatifs les épaississants non polymères, les agents nacrants, les opacifiants, les colorants ou les pigments, les parfums, les huiles minérales, végétales ou synthétiques, les cires, les vitamines, les filtres UV, les agents anti-radicalaires, les antipelliculaires, les conservateurs, les agents de stabilisation de pH, les solvants, et leurs mélanges.

L'homme du métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions peuvent être conditionnées sous diverses formes notamment dans des flacons, des flacons-pompe ou dans des récipients aérosols afin d'assurer une application de la composition sous forme vaporisée ou sous forme de mousse. Les compositions peuvent également imprégner des applicateurs, notamment des gants ou des lingettes.

La présente invention concerne également un procédé de lavage des matières kératiniques qui consiste à appliquer une quantité efficace d'une composition telle que décrite ci-dessus, sur les matières kératiniques, à effectuer un rinçage par exemple avec de l'eau après un éventuel temps de pose.

Les exemples suivants sont donnés à titre illustratif de la présente invention. Toutes les quantités indiquées sont exprimées en % en poids, sauf indication contraire.

### Exemples 1

On a préparé la composition de shampooing suivante :

| Composition | | Invention |
|---|---|---|
| Lauryléthersulfate de sodium (C12/C14 à 70/30) à 2.2 moles d'oxyde d'éthylène (TEXAPON AOS 225 UP de COGNIS) | | 4,9 g MA |
| Laurylsulfate de sodium (C12/C14 à 70/30) (TEXAPON LS 35 de COGNIS) | | 2,6 gMA |
| Cocoamidopropyl bétaïne (DEHYTON PK 45 de COGNIS) | | 3,05 g MA |
| 2-octyl dodécanol | | 2 g |
| PEG-136/Steareth-100/HDI Copolymer (RHEOLATE FX1100 de ELEMENTIS) | | 2 g |
| Polyquaternium-10 (POLYMER JR400 LT de AMERCHOL) | | 0,4 g |
| Glycérine | | 0,5 g |
| Conservateurs, parfum | | q,s, |
| Agent de pH | q.s. | pH 5,3 |
| Eau déminéralisée | q.s.p. | 100 g |

| | | |
|---|---|---|
| * : en Matière active (MA) | | |

La composition conforme à l'invention de l'exemple 1 est épaissie et a un aspect homogène et lisse, contrairement à une composition comparative ne comprenant pas de Rhéolate FX1100 qui est fluide.

La composition a été appliquée sur des cheveux décolorés préalablement mouillés (1 g par mèche de 2,7 g de cheveux). On a fait mousser le shampooing, on a laissé pauser 5 minutes puis on a rincé à l'eau.

Les cheveux humides et séchés, traités avec la composition de l'exemple 1 sont lisses et faciles à démêler.

### Exemples 2

On prépare la composition de shampooing suivante :

| Composition | | 2 |
|---|---|---|
| Lauryléthersulfate de sodium (C12/C14 à 70/30) à 2.2 moles d'oxyde d'éthylène (TEXAPON AOS 225 UP de COGNIS) | | 4,9 g MA |
| Laurylsulfate de sodium (C12/C14 à 70/30) (TEXAPON LS 35 de COGNIS) | | 2,6 gMA |
| Cocoamidopropyl bétaïne (DEHYTON PK 45 de COGNIS) | | 3,05 g MA |
| Alcool oléique | | 1,5 g |
| PEG-136/Steareth-100/HDI Copolymer (RHEOLATE FX1100 de ELEMENTIS) | | 2 g |
| Polyquaternium-10 (POLYMER JR400 LT de AMERCHOL) | | 0,4 g |
| Glycérine | | 0,5 g |
| Conservateurs, parfum | | q,s,* |
| Agent de pH | q.s. | pH 5,3 |
| Eau déminéralisée | q.s.p. | 100 g |

| | | |
|---|---|---|
| * : en Matière active (MA) | | |

### Exemples 3

On prépare la composition de shampooing suivante :

| Composition | | 2 |
|---|---|---|
| Lauryléthersulfate de sodium (C12/C14 à 70/30) à 2.2 moles d'oxyde d'éthylène (TEXAPON AOS 225 UP de COGNIS) | | 4,9 g MA |
| Laurylsulfate de sodium (C12/C14 à 70/30) (TEXAPON LS 35 de COGNIS) | | 2,6 gMA |
| Cocoamidopropyl bétaïne (DEHYTON PK 45 de COGNIS) | | 3,05 g MA |
| 2 décyl-1- tétradécanol | | 1,5 g |
| PEG-136/Steareth-100/HDI Copolymer (RHEOLATE FX1100 de ELEMENTIS) | | 2 g |
| Polyquaternium-10 (POLYMER JR400 LT deAMERCHOL) | | 0,4 g |
| Glycérine | | 0,5 g |
| Conservateurs, parfum | | q,s, |
| Agent de pH | q.s. | pH 5,3 |
| Eau déminéralisée | q.s.p. | 100 g |

| | | |
|---|---|---|
| * : en Matière active (MA) | | |

### Exemples 4 et 5

On a préparé la composition de shampooing suivante :

| Composition | | 4 | | 5 |
|---|---|---|---|---|
| Lauryléthersulfate de sodium (C12/C14 à 70/30) à 2.2 moles d'oxyde d'éthylène (TEXAPON AOS 225 UP de COGNIS) | | 4,9 | g MA | 4,9 g MA |
| Laurylsulfate de sodium (C12/C14 à 70/30) (TEXAPON LS 35 de COGNIS) | | 4 | gMA | 4 gMA |
| Disodium cocoamphodiacétate (MIRANOL C2M CONC MP deRHODIA)) | | 1,3 | g MA | 1,3 g MA |
| 2-octyl dodécanol | | 1,5 | g | |
| Decyl tetradécanol | | | | 1 g |
| PEG-136/Steareth-100/HDI Copolymer (RHEOLATE FX1100 de ELEMENTIS) | | 2 | g | 2 g |
| Polyquaternium-10 (POLYMER JR400 LT deAMERCHOL) | | 0,4 | g | 0,4 g |
| Conservateurs, parfum | | q.s. | | q.s. |
| Agent de pH | q.s. | pH 5,3 | | pH 5,3 |
| Eau déminéralisée | q.s.p. | 100 | g | 100 g |

| | | | | |
|---|---|---|---|---|
| * : en Matière active (MA) | | | | |

La composition conforme à l'invention des l'exemples 4 et 5 sont épaissies et a un aspect homogène et lisse, contrairement à une composition comparative ne comprenant pas de Rhéolate FX1100 qui est fluide.

La composition a été appliquée sur des cheveux décolorés préalablement mouillés (1 g par mèche de 2,7 g de cheveux). On a fait mousser le shampooing, on a laissé pauser 5 minutes puis on a rincé à l'eau.

Les cheveux humides et séchés, traités avec les compositions des exemples 4 et 5 sont lisses et faciles à démêler.

### Exemples 6 et 7

On prépare les compositions de shampooing suivantes :

| | | 6 | 7 |
|---|---|---|---|
| Lauryléthersulfate de sodium (C12/C14 à 70/30) à 2.2 moles d'oxyde d'éthylène (TEXAPON AOS 225 UP de COGNIS) | | 4,9 g MA | 4,9 g MA |
| Laurylsulfate de sodium (C12/C14 à 70/30) (TEXAPON LS 35 de COGNIS) | | 4 gMA | 4 gMA |
| Disodium cocoamphodiacétate (MIRANOL C2M CONC MP deRHODIA)) | | 1,3 g MA | 1,3 g MA |
| Gomme de guar cationique (JAGUAR C162 de RHODIA) | | 0,15 g | |
| Decyl-2 tetradécanol | | 0,05 g | 0,05 g |
| PEG-136/Steareth-100/HDI Copolymer (RHEOLATE FX1100 de ELEMENTIS) | | 2 g | 2 g |
| Polyquaternium-10 (UCARE POLYMER JR400 LT de AMERCHOL) | | | 0,1 g |
| Conservateurs, parfum | | q.s. | q.s. |
| Agent de pH | q.s. | pH 5,3 | pH 5,3 |
| Eau déminéralisée | q.s.p. | 100 g | 100 g |

| | | | |
|---|---|---|---|
| * : en Matière active (MA) | | | |

## Revendications

1. Composition de lavage des matières kératiniques, **caractérisée par le fait qu'**elle comprend, dans un milieu cosmétiquement acceptable :
- de 3 à 50% en poids par rapport au poids total de la composition d'un ou plusieurs tensioactifs anioniques,
- un ou plusieurs alcools liquides en C₁₀-C₃₀ choisis parmi les alcools en C₁₀-C₃₀ ramifiés et /ou insaturés, et
- un ou plusieurs polymères associatifs non ioniques,
la composition comprenant un ou plusieurs tensioactifs anioniques choisis parmi les alkylsulfates, et les alkyléthersulfates, en particulier sous forme de sels de métaux alcalins ou alcalino-terreux, d'ammonium, d'amine ou d'aminoalcool ; et les acides (alkyl en C₆₋₂₄)éther-carboxyliques polyoxyalkylénés et leurs sels, en particulier ceux comportant de 2 à 50 motifs oxyde d'éthylène ; et
ladite composition comprenant un ou plusieurs polymères associatifs non ioniques choisis parmi, seuls ou en mélanges :
- (1) les celluloses modifiées par des groupements comportant au moins une chaîne grasse comportant au moins 8 atomes de carbone; et
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

2. Composition selon la revendication précédente, **caractérisée en ce que** lesdits alcools en C₁₀-C₃₀ comportent de 12 à 30 atomes de carbone.

3. Composition selon la revendication précédente, **caractérisée en ce que** les alcools en C₁₀-C₃₀ présentent la structure R-OH, dans laquelle R désigne un groupement alkyle ramifié en C12-C24 ou alkényle en C12-C24.

4. Composition selon la revendication précédente, **caractérisée en ce que** les alcools en C₁₀-C₃₀ sont choisis parmi l'alcool oléique, l'alcool isocétylique, l'alcool isostéarylique, le 2-octyl-1-dodécanol, le 2-butyl octanol, le 2-hexyl-1-décanol, le 2-decyl-1-tétradécanol, le 2-tétradécyl-1-cétanol et leur(s) mélange(s).

5. Composition selon l'une quelconque des revendications précédentes, **caractérisée en ce que** le ou les alcools en C₁₀-C₃₀ liquides sont présents en une quantité allant de 0,01 à 10% en poids par rapport au poids total de la composition.

6. Composition selon l'une quelconque des revendications précédentes, **caractérisée en ce que** le ou les polymères associatifs non ioniques sont choisis parmi les polyéthers polyuréthanes susceptibles d'être obtenu par polycondensation d'au moins trois composés comprenant (i) au moins un polyéthylèneglycol comprenant de 100 à 180 moles d'oxyde d'éthylène, en particulier de 120 à 160 moles d'oxyde d'éthylène, (ii) un alcool gras en C16- 20 polyoxyéthyléné de 80 à 120 moles d'oxyde d'éthylène, en particulier 90 à 110 moles et notamment un alcool stéarylique polyoxyéthyléné comprenant de 80 à 120 moles d'oxyde d'éthylène, en particulier 90 à 110 moles et (iii) un diisocyanate aliphatique.

7. Composition selon l'une quelconque des revendications précédentes, **caractérisée en ce que** le ou les polymères associatifs non ioniques sont présents en une quantité allant de 0,1 à 5 % en poids, préférentiellement de 1,5 à 4% en poids par rapport au poids total de la composition.

8. Composition selon l'une quelconque des revendications précédentes, **caractérisée en ce qu'**elle comprend en outre au moins une tensioactif non ionique et/ou au moins un tensioactif amphotère.

9. Composition selon l'une quelconque des revendications précédentes, **caractérisée en ce que** le milieu cosmétiquement acceptable est constitué d'eau, ou d'un mélange d'eau et d'au moins un solvant organique.

10. Composition selon l'une quelconque des revendications précédentes, **caractérisée en ce que** la composition comprend en outre au moins un polymère cationique.

11. Composition selon l'une quelconque des revendications précédentes, **caractérisée en ce que** la composition comprend en outre au moins une silicone.

12. Utilisation de la composition selon l'une quelconque des revendications précédentes, pour le lavage et le conditionnement des matières kératiniques et en particulier des fibres kératiniques.

13. Procédé de lavage et de conditionnement des matières kératiniques et en particulier des fibres kératiniques, comprenant l'application d'une quantité efficace d'une composition selon l'une quelconque des revendications 1 à 11, sur les cheveux, et un rinçage après un éventuel temps de pose.

## Patentansprüche

1. Zusammensetzung zum Waschen von Keratinmaterialien, **dadurch gekennzeichnet, dass** sie in einem kosmetisch unbedenklichen Medium Folgendes umfasst:
- 3 bis 50 Gew.-%, bezogen auf das Gesamtgewicht der Zusammensetzung, eines oder mehrerer anionischer Tenside,
- einen oder mehrere flüssige C₁₀-C₃₀-Alkohole, die aus verzweigten und/oder ungesättigten C₁₀-C₃₀-Alkoholen ausgewählt sind, und
- ein oder mehrere nichtionische Assoziativpolymere,
wobei die Zusammensetzung ein oder mehrere anionische Tenside, die aus Alkylsulfaten, Alkylethersulfaten, insbesondere in Form von Alkali- oder Erdalkalimetall-, Ammonium-, Amin- oder Aminoalkoholsalzen, und polyoxyalkylenierten (C₆-₂₄-Alkyl)ethercarbonsäuren und Salzen davon, insbesondere denjenigen mit 2 bis 50 EthylenoxidEinheiten, ausgewählt sind, umfasst und
wobei die Zusammensetzung ein oder mehrere nichtionische Assoziativpolymere, die, alleine oder als Mischungen, aus:
- (1) Cellulosen, die mit Gruppen mit mindestens einer Fettkette mit mindestens 8 Kohlenstoffatomen modifiziert sind; und
- (6) Polyurethanpolyethern, die in ihrer Kette sowohl hydrophile Blöcke, die meist vom Polyoxyethylen-Typ sind, als auch hydrophobe Blöcke, bei denen es sich um aliphatische Ketten alleine und/oder cycloaliphatische und/oder aromatische Ketten handeln kann, enthalten; ausgewählt sind, umfasst.

2. Zusammensetzung nach dem vorhergehenden Anspruch, **dadurch gekennzeichnet, dass** die C₁₀-C₃₀-Alkohole 12 bis 30 Kohlenstoffatome enthalten.

3. Zusammensetzung nach dem vorhergehenden Anspruch, **dadurch gekennzeichnet, dass** die C₁₀-C₃₀-Alkohole die Struktur R-OH aufweisen, wobei R für eine verzweigte C₁₂-C₂₄-Alkyl- oder C₁₂-C₂₄-Alkenylgruppe steht.

4. Zusammensetzung nach dem vorhergehenden Anspruch, **dadurch gekennzeichnet, dass** die C₁₀-C₃₀-Alkohole aus Oleylalkohol, Isocetylalkohol, Isostearylalkohol, 2-Octyl-1-dodecanol, 2-Butyloctanol, 2-Hexyl-1-decanol, 2-Decyl-1-tetradecanol, 2-Tetradecyl-1-cetanol und Mischungen davon ausgewählt sind.

5. Zusammensetzung nach einem der vorhergehenden Ansprüche, **dadurch gekennzeichnet, dass** der flüssige C₁₀-C₃₀-Alkohol bzw. die flüssigen C₁₀-C₃₀-Alkohole in einer Menge im Bereich von 0,01 bis 10 Gew.-%, bezogen auf das Gesamtgewicht der Zusammensetzung, vorliegt bzw. vorliegen.

6. Zusammensetzung nach einem der vorhergehenden Ansprüche, **dadurch gekennzeichnet, dass** das nichtionische Assoziativpolymer bzw. die nichtionischen Assoziativpolymere aus Polyurethanpolyethern, die durch Polykondensation von mindestens drei Verbindungen, die (i) mindestens ein Polyethylenglykol mit 100 bis 180 mol Ethylenoxid, insbesondere 120 bis 160 mol Ethylenoxid, (ii) einen mit 80 bis 120 mol und insbesondere 90 bis 110 mol Ethylenoxid polyoxyethylenierten C₁₆₋₂₀-Fettalkohol und insbesondere einen polyoxyethylenierten Stearylalkohol mit 80 bis 120 mol und insbesondere 90 bis 110 mol Ethylenoxid und (iii) ein aliphatisches Diisocyanat umfassen, erhältlich sind, ausgewählt ist bzw. sind.

7. Zusammensetzung nach einem der vorhergehenden Ansprüche, **dadurch gekennzeichnet, dass** das nichtionische Assoziativpolymer bzw. die nichtionischen Assoziativpolymere in einer Menge im Bereich von 0,1 bis 5 Gew.-%, vorzugsweise von 1,5 bis 4 Gew.-%, bezogen auf das Gesamtgewicht der Zusammensetzung, vorliegt bzw. vorliegen.

8. Zusammensetzung nach einem der vorhergehenden Ansprüche, **dadurch gekennzeichnet, dass** sie außerdem mindestens ein nichtionisches Tensid und/oder mindestens ein amphoteres Tensid umfasst.

9. Zusammensetzung nach einem der vorhergehenden Ansprüche, **dadurch gekennzeichnet, dass** das kosmetisch unbedenkliche Medium aus Wasser oder einer Mischung von Wasser und mindestens einem organischen Lösungsmittel besteht.

10. Zusammensetzung nach einem der vorhergehenden Ansprüche, **dadurch gekennzeichnet, dass** die Zusammensetzung außerdem mindestens ein kationisches Polymer umfasst.

11. Zusammensetzung nach einem der vorhergehenden Ansprüche, **dadurch gekennzeichnet, dass** die Zusammensetzung außerdem mindestens ein Silikon umfasst.

12. Verwendung der Zusammensetzung nach einem der vorhergehenden Ansprüche zum Waschen und Konditionieren von Keratinmaterialien und insbesondere Keratinfasern.

13. Verfahren zum Waschen und Konditionieren von Keratinmaterialien und insbesondere Keratinfasern, bei dem man eine wirksame Menge einer Verbindung nach einem der Ansprüche 1 bis 11 auf die Haare aufbringt und nach einer fakultativen Einwirkungszeit spült.

## Claims

1. Composition for washing keratin materials, **characterized in that** it comprises, in a cosmetically acceptable medium:
- from 3% to 50% by weight, relative to the total weight of the composition, of one or more anionic surfactants,
- one or more C₁₀-C₃₀ liquid alcohols chosen from branched and/or unsaturated C₁₀-C₃₀ alcohols, and
- one or more nonionic associative polymers,
the composition comprising one or more anionic surfactants chosen from alkyl sulfates and alkyl ether sulfates, in particular in the form of alkali metal or alkaline-earth metal salts, ammonium salts, amine salts or amino alcohol salts; and polyoxyalkylenated (C₆₋₂₄)alkyl ether carboxylic acids and salts thereof, in particular those comprising from 2 to 50 ethylene oxide units; and
the said composition comprising one or more nonionic associative polymers chosen from, alone or as mixtures:
- (1) celluloses modified with groups comprising at least one fatty chain comprising at least 8 carbon atoms; and
- (6) polyurethane polyethers comprising in their chain both hydrophilic blocks usually of polyoxyethylenated nature and hydrophobic blocks, which may be aliphatic sequences alone and/or cycloaliphatic and/or aromatic sequences.

2. Composition according to the preceding claim, **characterized in that** the said C₁₀-C₃₀ alcohols comprises from 12 to 30 carbon atoms.

3. Composition according to the preceding claim, **characterized in that** the C₁₀-C₃₀ alcohols have the structure R-OH, in which R denotes a C₁₂-C₂₄ branched alkyl or C₁₂-C₂₄ alkenyl group.

4. Composition according to the preceding claim, **characterized in that** the C₁₀-C₃₀ alcohols are chosen from oleyl alcohol, isocetyl alcohol, isostearyl alcohol, 2-octyl-1-dodecanol, 2-butyloctanol, 2-hexyl-1-dodecanol, 2-decyl-1-tetradecanol and 2-tetradecyl-1-cetanol, and mixtures thereof.

5. Composition according to any one of the preceding claims, **characterized in that** the liquid C₁₀-C₃₀ alcohol(s) are present in an amount ranging from 0.01% to 10% by weight relative to the total weight of the composition.

6. Composition according to any one of the preceding claims, **characterized in that** the nonionic associative polymer(s) are chosen from polyurethane polyethers that may be obtained by polycondensation of at least three compounds comprising (i) at least one polyethylene glycol comprising from 100 to 180 mol of ethylene oxide and in particular from 120 to 160 mol of ethylene oxide, (ii) a C₁₆₋₂₀ fatty alcohol polyoxyethylenated with 80 to 120 and in particular 90 to 110 mol of ethylene oxide and especially a polyoxyethylenated stearyl alcohol comprising from 80 to 120 and in particular 90 to 110 mol of ethylene oxide, and (iii) an aliphatic diisocyanate.

7. Composition according to any one of the preceding claims, **characterized in that** the nonionic associative polymer(s) are present in an amount ranging from 0.1% to 5% by weight and preferentially from 1.5% to 4% by weight relative to the total weight of the composition.

8. Composition according to any one of the preceding claims, **characterized in that** it also comprises at least one nonionic surfactant and/or at least one amphoteric surfactant.

9. Composition according to any one of the preceding claims, **characterized in that** the cosmetically acceptable medium consists of water or of a mixture of water and of at least one organic solvent.

10. Composition according to any one of the preceding claims, **characterized in that** the composition also comprises at least one cationic polymer.

11. Composition according to any one of the preceding claims, **characterized in that** the composition also comprises at least one silicone.

12. Use of the composition according to any one of the preceding claims, for washing and conditioning keratin materials and in particular keratin fibres.

13. Process for washing and conditioning keratin materials and in particular keratin fibres, comprising the application of an effective amount of a composition according to any one of Claims 1 to 11 to the hair, and rinsing after an optional leave-on time.
